(19) Europäisches Patentamt
European Patent Office
Office européen des brevets

(11) Veröffentlichungsnummer: **0 293 753**
**A2**

(12) **EUROPÄISCHE PATENTANMELDUNG**

(21) Anmeldenummer: **88108379.4**

(22) Anmeldetag: **26.05.88**

(51) Int. Cl.⁴: **C07C 69/533 , C07C 67/333 , C07C 57/03 , C07C 51/09**

(30) Priorität: **02.06.87 DE 3718801**

(43) Veröffentlichungstag der Anmeldung: **07.12.88 Patentblatt 88/49**

(84) Benannte Vertragsstaaten: **AT BE CH DE ES FR GB GR IT LI LU NL SE**

(71) Anmelder: **Desitin Arzneimittel GmbH**
**Weg beim Jäger 214**
**D-2000 Hamburg 63(DE)**

(72) Erfinder: **Schäfer, Helmut**
**Stegenerweg 40**
**D-2061 Kayhude(DE)**

(74) Vertreter: **UEXKÜLL & STOLBERG**
**Patentanwälte**
**Beselerstrasse 4**
**D-2000 Hamburg 52(DE)**

(54) **Verfahren zur Herstellung von E-2-Propyl-2-pentensäure und physiologisch verträglichen Salzen derselben.**

(57) Es wird ein neues Verfahren zur Herstellung von E-2-Propyl-2-pentensäure sowie von deren physiologisch verträglichen Salzen beschrieben, bei dem 2-Brom-2-propylpentansäure-ethylester als Ausgangsverbindung verwendet wird. Das Brom wird mit einem ringförmigen tertiären Amin in Acetonitril als Lösungsmittel abgespalten, wobei bevorzugt das E-Isomere des Ethylesters gebildet wird. Die freie Säure wird durch anschließende Verseifung unter schonenden Bedingungen erhalten und gegebenenfalls vorzugsweise unter Verwendung der entsprechenden Salze der Kohlensäure in wäßriger Acetonlösung in die Salzform überführt.

EP 0 293 753 A2

Xerox Copy Centre

## Verfahren zur Herstellung von E-2-Propyl-2-pentensäure und physiologisch verträglichen Salzen derselben

E-2-Propyl-2-pentensäure, ein Derivat der Valproinsäure, hat in neuerer Zeit großes Interesse als mögliches Therapeutikum zur Behandlung der Epilepsie hervorgerufen. Während nämlich unter einer konventionellen Behandlung mit Valproinsäure in Einzelfällen hepatotoxische und teratogene Eigenschaften zu befürchten sind, zeigen vergleichende Tierversuche mit E-2-Propyl-2-pentensäure, daß diese bei mindestens gleichwertiger therapeutischer Wirksamkeit keine lebertoxischen oder teratogenen Effekte hervorbringt (vgl. W. Löscher, Drugs of the Future, 10, 389-391 (1985)). Die toxikologisch günstigen Eigenschaften wurden speziell bei dem E-Isomeren der 2-Propylpenten-2-säure beobachtet.

Die E-2-Propyl-2-pentensäure ist eine kristalline Verbindung, deren Schmelzpunkt bei 38 bis 40 ° C liegt und die als solche bereits seit langem bekannt ist.

Ein Weg zur Herstellung der Verbindung führt nach einem bekannten Verfahren von der 2-Brom-2-propylpentansäure oder einem Derivat derselben als Ausgangsprodukte über die Abspaltung von HBr mittels einer alkalischen Verbindung, vorzugsweise einem tertiären Amin bei Temperaturen oberhalb 100 ° C zur 2-Propyl- 2-pentensäure. Ein Nachteil dieses Verfahrens liegt darin, daß bei der Abspaltung von HBr wechselnde Mengen der Z- und E-Isomeren entstehen, welche meistens zusätzlich mit durch Wanderung der Doppelbindung entstandener 2-Propyl-3-pentensäure verunreinigt sind.

Es ist demgemäß Aufgabe der Erfindung, ein Verfahren zu schaffen, bei dem die Bildung des E-Isomeren der 2-Propyl-2-pentensäure begünstigt ist.

Zur Lösung der Aufgabe wird das Verfahren gemäß Anspruch 1 vorgeschlagen.

Überraschenderweise hat es sich gezeigt, daß bevorzugt das E-Isomere des 2-Propyl-2-pentensäure-ethylesters gebildet wird, wenn die Abspaltung des Broms aus der Ausgangsverbindung 2-Brom-2-propylpentansäure-ethylester gemäß den nachfolgenden Formeln II und III

II III

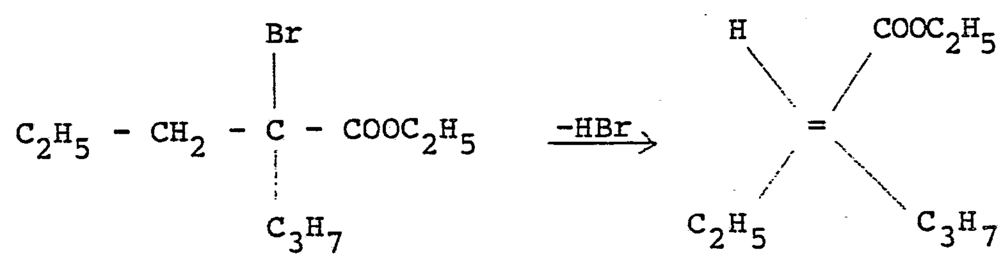


unter Verwendung eines cyclischen tertiären Amins und in Gegenwart von Acetonitril als Lösungsmittel vorgenommen wird. Vorzugsweise wird erfindungsgemäß Triethylendiamin als ringförmiges tertiäres Amin verwendet.

Die Abspaltung wird durch Kochen des Reaktionsgemisches am Rückfluß über mehrere Stunden, vorzugsweise 6 bis 10 Stunden vorgenommen. Beim Einsatz von Triethylendiamin als tertiäres Amin fällt während des Kochens das HBr-Salz dieses Amins kristallin aus und kann zur Rückgewinnung des Triethylendiamins abgetrennt werden.

Ferner wird vor der üblichen Aufarbeitung des Reaktionsgemisches ein großer Teil des eingesetzten Acetonitrils durch Abdestillation zurückgewonnen. Der Rückstand wird in Wasser gelöst und nach Ansäuern wird der Ester unter Einsatz eines organischen Lösungsmittels abgetrennt und durch Destillation gereinigt.

Vorzugsweise wird die Abtrennung des E-Isomeren beispielsweise durch Feindestillation in diesem Verfahrensstadium vorgenommen. Die Abtrennung kann jedoch auch im Anschluß an die nachfolgend erläuterte Umsetzung in die freie Säure erfolgen.

Zur Herstellung der freien Säure wird der Ester schonend verseift. Dies kann beispielsweise bei Zimmertemperatur mit Natriumhydroxid in Methanol als Lösungsmittel geschehen. Man läßt den Ansatz einige Tage lang, vorzugsweise 4 bis 6 Tage lang stehen und gewinnt nach Ansäuern der Lösung die freie Säure in üblicher Weise, beispielsweise durch Destillieren unter Rückgewinnung des Methanols. Geringe Mengen von E-2-Propyl-3-pentensäure können nach üblichen Verfahren, beispielsweise durch Feindestillation von dem gewünschten Derivat abgetrennt werden.

Die freie Säure kann, falls gewünscht, in üblicher Weise in die Form eines physiologisch verträglichen Salzes überführt werden.

Gemäß einer besonders bevorzugten Ausführungsform wird die Umsetzung in wäßrigem Aceton als Lösungsmittel in Gegenwart des entsprechenden Salzes der Kohlensäure durchgeführt. Soll beispielsweise das Natriumsalz der E-2-Propyl-2-pentensäure hergestellt werden, so wird vorzugsweise Natriumcarbonat in einer Lösung der freien Säure in einem Aceton/Wassergemisch einige Stunden lang am Rückfluß gekocht. Nach Abkühlen auf eine Temperatur unterhalb 0°C wird dann das Natriumsalz als Niederschlag feiner weißer Kristalle erhalten.

Die Erfindung wird nachfolgend anhand von Beispielen erläutert.

## Beispiel 1

Herstellung von 2-Propyl-2-pentensäure-ethylester

Zu einer Lösung von 100 g 2-Brom-2-propylpentansäure-ethylester in 200 ml Acetonitril wurde eine Lösung von 100 g Triethylendiamin (l,4-Diazabicyclo[2,2,2]octan) in 400 ml Acetonitril gegeben. Das farblose Reaktionsgemisch wurde 8 Stunden lang am Rückfluß gekocht. Bereits nach 15 Minuten begann die Ausfällung eines weißen Salzes. Anschließend wurden etwa 450 ml Acetonitril am Rotationsverdampfer bei 200 mbar und 40°C abdestilliert und zurückgewonnen. Der ölig-kristalline Rückstand wurde mit 1000 ml Wasser versetzt, wobei sich das ausgeschiedene Salz löste. Nach Ansäuern mit 1 N Salzsäure auf pH 1 wurde das ausgeschiedene Öl in 150 ml n-Hexan aufgenommen und die wäßrige Phase noch zweimal mit je 150 ml n-Hexan extrahiert. Nach Waschen und Trocknen der organischen Phase wurde destilliert. Die Ausbeute betrug 54,3 g eines Gemisches von E- und Z-2-Propyl-2-pentensure-ethylester mit einem Gehalt von 42 g des gesuchten E-Isomeren, entsprechend 62% bezogen auf das Ausgangsprodukt.

## Beispiel 2

Herstellung von 2-Propyl-2-pentensäure

10 g 2-Propyl-2-pentenethylester gemäß Beispiel 1 wurden zu einer Lösung von 20 g Natriumhydroxid in 150 ml Methanol gegeben und bei Zimmertemperatur stehengelassen. Nach 5 Tagen wurde der Ansatz unter Rückgewinnung des Methanols in üblicher Weise nach Ansäuren aufgearbeitet. Es wurde in nahezu quantitativer Ausbeute ein Gemisch von 2-Propyl-2-pen tensäure (E-Isomeres) und 2-Propyl-3-pentensäure (E-Isomeres) im Verhältnis von etwa 80 : 20 erhalten, das durch Feindestillation an einer wirksamen Kolonne getrennt wurde und E-2-Propyl-2-pentensäure mit $Kp_{15}$ 135-136°C lieferte.

## Beispiel 3

Herstellung des Natriumsalzes der E-2-Propyl-2-pentensäure

10 g feingemahlenes wasserfreies Natriumcarbonat wurden in einer Lösung von 142,2 g E-2-Propyl-2-pentensäure in 1600 ml Aceton und 54 ml Wasser 6 Stunden lang unter Rückfluß gekocht und die Lösung anschließend filtriert. Aus dem Filtrat kristallisierte nach Abkühlen auf etwa -5 ° C das Natriumsalz in feinen, weißen Kristallen aus. Die Ausbeute nach Aufarbeitung der Mutterlauge war nahezu quantitativ. Der Schmelzpunkt der gebildeten Kristalle lag über 300 ° C. Das erhaltene Salz war leicht wasserlöslich.

## Ansprüche

1. Verfahren zur Herstellung von E-2-Propyl-2-pentensäure der Formel I

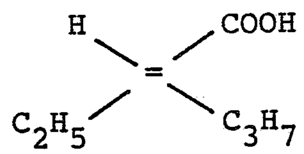

I

oder physiologisch verträglichen Salzen derselben durch Abspalten von Brom aus 2-Brom-2-propylpentansäure-ethylester der Formel II

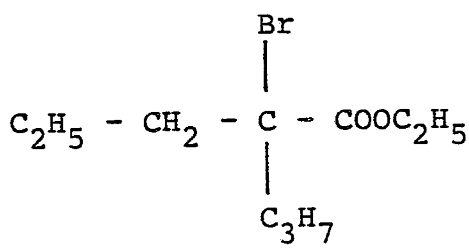

II

als Ausgangsverbindung bei erhöhter Temperatur unter Verwendung eines tertiären Amins, Umsetzen des Esters zur Säure unter schonenden Bedingungen und gegebenenfalls Umsetzen der Säure in ein physiologisch verträgliches Salz derselben, **dadurch gekennzeichnet,** daß man als tertiäres Amin ein ringförmiges Amin verwendet und die Abspaltung des Broms in Acetonitril als Lösungsmittel durchführt.

2. Verfahren nach Anspruch 1, **dadurch gekennzeichnet,** daß man als ringförmiges tertiäres Amin Triethylendiamin verwendet.

3. Verfahren nach einem der Ansprüche 1 oder 2, **dadurch gekennzeichnet,** daß man zur Herstellung eines physiologisch verträglichen Salzes der E-2-Propyl-2-pentensäure die freie Säure mit dem korrespondierenden Salz der Kohlensäure in einer Mischung aus Aceton und Wasser als Lösungsmittel umsetzt.

4. Verfahren nach Anspruch 3, **dadurch gekennzeichnet,** daß man als Salz der Kohlensäure Natriumcarbonat verwendet.